# FASCICULE DE BREVET EUROPEEN

(11) **EP 1 113 867 B1**
(45) Date de publication et mention de la délivrance du brevet: **29.10.2003**
(21) Numéro de dépôt: 99940253.0
(22) Date de dépôt: 30.08.1999
(51) Int. Cl.: B01J 8/04, B01D 15/02

(54) **DISTRIBUTEUR-MELANGEUR-EXTRACTEUR DE FLUIDES ET PROCEDE ASSOCIE**
VERTEILER-MISCHER-ABSCHEIDER VON FLUIDEN SOWIE VERFAHREN DAFÜR
FLUID DISTRIBUTOR-MIXER-EXTRACTOR AND RELATED METHOD

(30) Priorité: 02.09.1998 FR 9810996
(43) Date de publication de la demande: 11.07.2001
(73) Titulaire: Institut Français du Pétrole, 92852 Rueil-Malmaison Cedex (FR)
(72) Inventeur: FERSCHNEIDER, Gilles, F-69360 Saint Symphorien d'Ozon (FR); VIGUIE, Jean-Christophe, D-69360 Saint Symphorien d'Ozon (FR); CALLEBERT, Olivier, F-92500 Rueil-Malmaison (FR); HOFFMANN, Frédéric, F-75010 Paris (FR)
(86) Numéro de dépôt international: FR9902065
(87) Numéro de publication internationale: WO00013782

(56) Documents cités:
- EP-A- 0 769 316
- WO-A-95/03867
- US-A- 3 723 300

## Description

La présente invention concerne un dispositif, ou en abrégé DME, et un procédé permettant de collecter, distribuer, mélanger, additionner et/ou soutirer plusieurs fluides (au moins un fluide principal et au moins deux fluides secondaires).

L'invention trouve notamment son application dans le domaine de la chromatographie pour des fluides dans un état gazeux, liquide ou supercritique.

L'invention concerne un DME pouvant être utilisé dans un procédé de séparation en lit mobile simulé de paraxylène contenu dans un mélange de xylènes et d'éthylbenzène, en vue de la synthèse d'acide téréphtalique, un intermédiaire pétrochimique dans la fabrication des textiles.

L'invention peut aussi être utilisée pour séparer, par exemple, un mélange d'isomères de xylène et d'éthylbenzène, un mélange d'un composé sélectionné à partir d'acides gras saturés et leurs esters, un mélange de paraffine et d'oléfines, un mélange d'isoparaffines et de normal paraffines, et d'autres composés.

Le dispositif selon l'invention peut opérer en phase liquide, en phase vapeur ou en phase supercritique et dans tous les domaines de séparation notamment en chimie, pétrochimie ou pétrolier.

Dans le domaine des procédés de séparation, il est habituel de faire appel à des systèmes de lit mobile simulé pour séparer les corps comportant par exemple au moins deux composés chimiques différents ou encore deux isomères d'un même composé. Le matériau d'adsorption utilisé est par exemple un solide.

L'arrière plan technologique illustrant la mise en oeuvre d'un dispositif d'adsorption à contre-courant simulé est décrit par exemple dans le brevet US-2.985.589.

Dans ces procédés, un fluide principal introduit par une pompe s'écoule à travers le lit de solide le long de l'axe central de la colonne. Pour obtenir les meilleures performances de ce procédé, il est important que le fluide principal s'écoule à travers l'adsorbant selon un écoulement de type piston (plug flow), et ainsi d'avoir une composition et un front d'écoulement le plus uniforme possible en tous points de la surface du lit d'adsorbant.

L'art antérieur décrit différents moyens tentant d'obtenir et de conserver un tel écoulement.

Pour des applications à contre-courant simulé, le dispositif décrit dans le brevet US-3.214.247, montre une structure comportant une grille supérieure, une grille inférieure de maintien des particules et deux baffles horizontaux non perforés positionnés entre ces deux grilles. Les fluides sont ajoutés ou extraits au niveau d'un espace central entre les baffles ou déflecteurs par un conduit traversant la section totale du dispositif. Un tel dispositif permet de remélanger le fluide principal en écoulement dans la colonne et aussi d'assurer un bon mélange d'un fluide additionné au fluide principal.

On peut aussi mentionner les deux brevets US-5.792.346 et US-5.755.960 qui divulguent des panneaux de distribution de fluides ou DME, ayant notamment pour fonction de mélanger, extraire ou additionner des fluides. Ces DME sont reliés à des circuits de distribution-collecte des fluides, ayant pour fonction notamment d'homogénéiser le temps de parcours des particules du fluide, de l'extérieur de la colonne jusqu'aux panneaux et inversement d'un panneau vers un réseau de collecte extérieur, par exemple.

En effet, la dispersion dans la composition de l'écoulement et dans le temps de parcours des particules de fluides peut aussi provenir de la façon dont sont distribués ou extraits les fluides jusqu'aux DME ou à partir des DME.

Certains circuits de distribution ou de collecte sont adaptés pour diminuer le temps de dispersion des fluides. La géométrie de ces circuits est en règle générale adaptée à la géométrie des plateaux et à la disposition des DME au niveau de ces plateaux.

Par exemple, dans le brevet US US-5.792.346, le circuit de distribution ou d'extraction des fluides secondaires montre une symétrie de distribution et une isolongueur des lignes de transfert des fluides. Ces circuits permettent une distribution des fluides de type râteau ou une distribution radiale à partir ou vers le centre de la colonne de séparation.

Dans le brevet US-5.755.960, le circuit de distribution-collecte est composé de plusieurs conduites radiales comportant plusieurs ramifications pour distribuer ou collecter des fluides secondaires vers ou à partir de chaque panneau formant un plateau de distribution. Les ramifications sont réparties sur la totalité ou sur une partie de la longueur du conduit d'alimentation radial auxquelles elles sont raccordées. Une autre variante consiste à distribuer les fluides à partir d'une couronne ou de demi-couronnes positionnées à la périphérie de la colonne. Les conduits de transfert des fluides jusqu'à un DME sont répartis sur toute la longueur de la couronne ou des demi-couronnes.

L'objet de la présente invention est de fournir un dispositif designé sous le terme DME ayant notamment pour fonction de collecter, mélanger, extraire ou remélanger des fluides, qui présente un agencement spécifique de deux chambres de mélange et d'un rail de distribution-collecte pour assurer une distribution ou une collecte la plus symétrique possible des fluides.

Il a pour objectif notamment d'améliorer le mélange des fluides et de fait la composition résultante. Il permet d'obtenir un écoulement de type piston et une composition de l'écoulement à travers l'adsorbant la plus homogène possible.

Dans toute la suite de la description, on désigne par chambre de niveau 1 une chambre qui a pour fonction de diviser au moins par deux un fluide ou de collecter deux flux de fluides, et chambre de niveau 2 une chambre qui assure la division au moins par deux d'un fluide issu d'une chambre de niveau 1 ou la collecte de deux flux de fluides pour les envoyer vers une chambre de niveau 1.

Sous le terme DME on désigne un panneau ayant notamment pour fonction de collecter, mélanger, extraire ou remélanger un ou plusieurs fluides.

La présente invention concerne un dispositif (DME en abrégé) ou panneau pour collecter, distribuer, mélanger ou soutirer plusieurs fluides, au moins un fluide principal et au moins deux fluides secondaires,
comportant au moins :
* des moyens de collecte d'un fluide principal,
* au moins deux rails d'injection et/ou de soutirage permettant le passage des fluides secondaires,
* au moins deux chambres de mélange qui communiquent avec les rails d'injection et/ou de soutirage à l'aide d'orifices ou des passages,
* des moyens de redistribution du fluide issu desdites chambres de mélange,
* des moyens (5a, 5b) de séparation des moyens de collecte et de redistribution.

Il est caractérisé en ce que les rails sont disposés l'un au-dessus de l'autre et en ce que lesdites chambres de mélange sont disposées de part et d'autre d'au moins un desdits rails.

Les chambres de mélange sont par exemple disposées par rapport aux orifices de manière à obtenir une injection ou un soutirage du ou des fluides la plus uniforme possible dans la ou les chambres de mélange.

Les rails d'injection et les chambres de mélange sont par exemple situés sensiblement au barycentre de la surface dudit DME.

Le rail supérieur peut être pourvu d'au moins deux conduits et le rail inférieur d'au moins un conduit, les conduits étant disposés de manière à assurer la circulation des fluides secondaires de la façon la plus symétrique dans les rails et les chambres de mélange.

Les ouvertures de passage d'un fluide secondaire du ou des rails ayant pour fonction d'injecter des fluides secondaires vers une chambre de mélange ont un axe tel que le fluide issu percute une partie au moins d'une paroi pleine d'un des éléments du DME.

Le circuit inférieur ou rail inférieur du fluide secondaire a sa paroi inférieure disposée, par exemple, au niveau de la grille inférieure (pour augmenter la tenue mécanique de l'ensemble).

La présente invention concerne aussi un dispositif ou une colonne de séparation comportant par exemple, une enceinte, l'enceinte comportant au moins un premier lit d'adsorbant (A₁) et au moins un deuxième lit d'adsorbant (A₂), les lits étant séparés par un plateau Pi comportant un ou plusieurs panneaux (DME) présentant l'une des caractéristiques des revendications 1 à 5, les DME étant en liaison avec l'extérieur par l'intermédiaire de conduits de raccordements.

Le rail supérieur d'un DME peut avoir une fonction de collecte des fluides et le rail inférieur d'un DME une fonction d'injection des fluides.

Le rail supérieur d'un DME a par exemple une fonction d'injection des fluides et le rail inférieur d'un DME une fonction de collecte des fluides.

Les rails supérieur et inférieur d'un DME peuvent avoir chacun une fonction d'injection-collecte des fluides.

Les ouvertures du ou des rails ayant une fonction d'injection sont par exemple disposées de façon telles que le jet de fluide passant à travers l'orifice percute au moins une partie d'une paroi pleine d'un des éléments du DME.

Les ouvertures des rails peuvent être disposées en alternance ou en quiconque.

Les paramètres des rails et des ouvertures sont par exemple choisis parmi les données suivantes :
* un diamètre des ouvertures compris, par exemple, entre 2 et 15 mm et de préférence entre 4 et 7 mm, les ouvertures pouvant avoir une fonction d'injection,
* un pas de perforation compris entre 25 et 400 mm et de préférence entre 50 et 200 mm,
* une vitesse de passage des fluides compris entre 3 - 20 m/s et de préférence entre 5 - 15 m/s, la valeur du pas considéré avec la valeur de la vitesse permet d'obtenir un bon mélange du fluide secondaire et du fluide principal.

Les paramètres des chambres de mélange et des ouvertures des chambres sont par exemple choisis parmi les valeurs suivantes :
* un diamètre des ouvertures compris entre 5 et 50 mm, et de préférence entre 10 et 25 mm,
* un pas de perforation choisi parmi l'intervalle 25 - 400 mm et de préférence dans l'intervalle 50 - 200 mm,
* une vitesse de passage du mélange compris entre 0.5 et 3.5 m/s et de préférence entre 1.0 - 2.0 m/s.

La présente invention concerne aussi un procédé permettant de séparer au moins un composé à partir d'un mélange ou d'un corps par adsorption. Il se caractérise en ce que l'on met en contact un fluide principal à partir duquel on cherche à séparer certains composés avec un adsorbant choisi en fonction de son pouvoir à séparer les composés, on injecte et/ou on extrait les fluides secondaires par l'intermédiaire d'un panneau ou DME pour collecter, distribuer, mélanger ou soutirer plusieurs fluides, au moins un fluide principal et au moins deux fluides secondaires,
comportant au moins :
* des moyens de collecte d'un fluide principal,
* au moins deux rails d'injection et/ou de soutirage permettant le passage des fluides secondaires,
* au moins deux chambres de mélange qui communiquent avec les rails d'injection ou de soutirage à l'aide d'orifices ou des passages,
* des moyens de redistribution du fluide issu desdites chambres de mélange,
* des moyens de séparation des espaces de collecte et de redistribution,
les rails étant disposés l'un au-dessus de l'autre et les chambres de mélange disposées de part et d'autre des rails.

On peut injecter un fluide de manière la plus uniforme possible dans les chambres de mélange en passant par les orifices ayant une fonction d'injection.

On peut regrouper les fluides par fonction (injection/ou soutirage) ou par nature ou par valeur de débit.

Le dispositif et le procédé selon l'invention sont bien adaptés pour séparer une charge en chromatographie pour des fluides dans un état gazeux, liquide ou supercritique.

Le dispositif et le procédé selon l'invention sont aussi bien adaptés à la séparation de paraxylène en lit mobile simulé.

Par rapport aux dispositifs de l'art antérieur, le DME selon l'invention et le dispositif de séparation comportant un ou plusieurs de ces DME présentent notamment les avantages suivants :
- du fait de la bonne symétrie de distribution des fluides secondaires et du fluide principal, le mélange est amélioré sur l'ensemble du plateau, ce qui confère à l'écoulement circulant dans le dispositif de séparation une composition plus homogène, et un écoulement général de type piston,
- la distribution ou l'extraction des fluides dans ou à partir d'un DME s'effectue à l'aide d'un système de distribution-collecte assurant une dispersion minimale entre les temps de parcours des flux de fluides pour un même plateau,
- une densité de flux sensiblement uniforme sur l'ensemble de la colonne,
- une distribution spatiale homogène des débits des fluides secondaires sur la surface du plateau.

D'autres caractéristiques et avantages du dispositif selon l'invention apparaîtront à la lecture de la description d'exemples donnés ci-après à titre illustratif et nullement limitatif en se référant aux dessins annexés où :
- la figure 1 représente de manière détaillée, un exemple de réalisation d'un panneau ou DME selon l'invention, les figures 1A, 1B et 1C correspondent à des vues en coupe et de dessus, de plusieurs variantes de réalisation,
- les figures 2A et 2B représentent une vue de dessus et une coupe d'un exemple d'alimentation du panneau,
- la figure 3 schématise une coupe longitudinale d'une colonne de séparation comportant plusieurs plateaux comportant des panneaux selon l'invention,
- les figures 4A, 4B et 4C représentent plusieurs variantes de réalisation d'un système de distribution-collecte des fluides.

L'exemple donné ci-après concerne, à titre illustratif et nullement limitatif, une application du DME selon l'invention disposé entre deux lits de solides granulaires ou lits d'adsorbants dans une colonne de séparation. La colonne de séparation est par exemple destinée à séparer du paraxylène. Les fluides dénommés " fluides secondaires " peuvent être la charge, l'extrait ou le raffinat obtenus par séparation ou encore le désorbant utilisé pour extraire des lits d'adsorbants les constituants qui ont été adsorbés lors du procédé de séparation.

Un panneau élémentaire ou DME ayant pour fonction notamment de collecter, mélanger, extraire ou remélanger des fluides est divisé à l'aide d'un rail de distribution-collecte, en deux surfaces sensiblement égales. Le rail de distribution-collecte est formé par exemple de la superposition de deux boîtes de section rectangulaire ou rails.

Un panneau ou DME comporte une grille supérieure 3 et une grille inférieure 4, en considérant le sens de circulation du fluide principal à l'intérieur de la colonne de séparation. La grille 3 supérieure assure au moins en partie la collecte du fluide principal, alors que la grille 4 inférieure assure au moins en partie la redistribution du mélange issu de la chambre de mélange sur l'ensemble du panneau et sur l'ensemble de la surface d'un lit d'adsorbant disposé en dessous.

Entre ces deux grilles, par exemple de type à fentes, sont disposés différents éléments :
* deux déflecteurs 5a, 5b, ou baffles qui ont notamment pour fonction de séparer le canal de collecte et le canal de distribution décrits ci-après,
* deux rails 6, 7 permettant le passage des fluides secondaires, ces rails sont disposés l'un au-dessus de l'autre, par exemple. Le rail supérieur 6 peut être situé au-dessus des déflecteurs, alors que le circuit inférieur 7 peut être positionné entre les deux déflecteurs 5a, 5b et se prolonger selon sa hauteur au-dessous des déflecteurs;

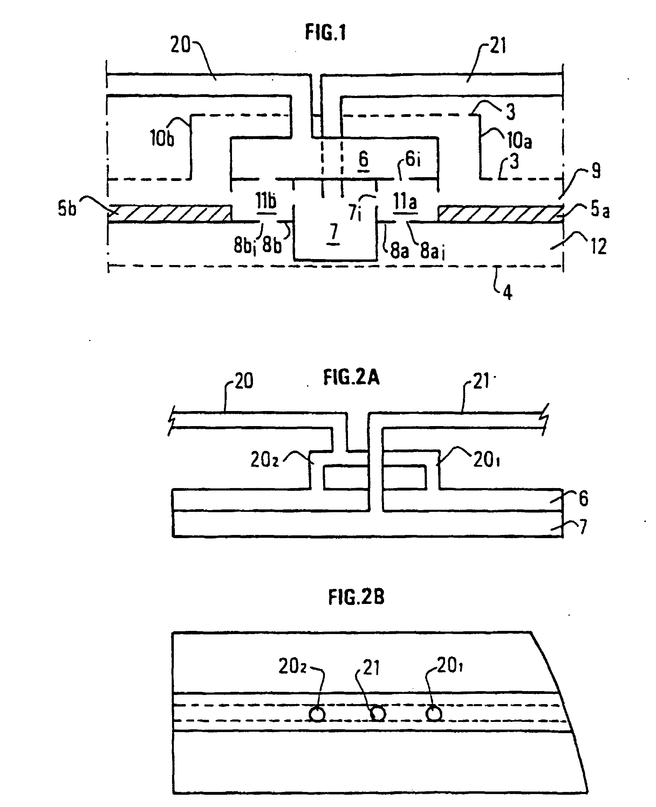

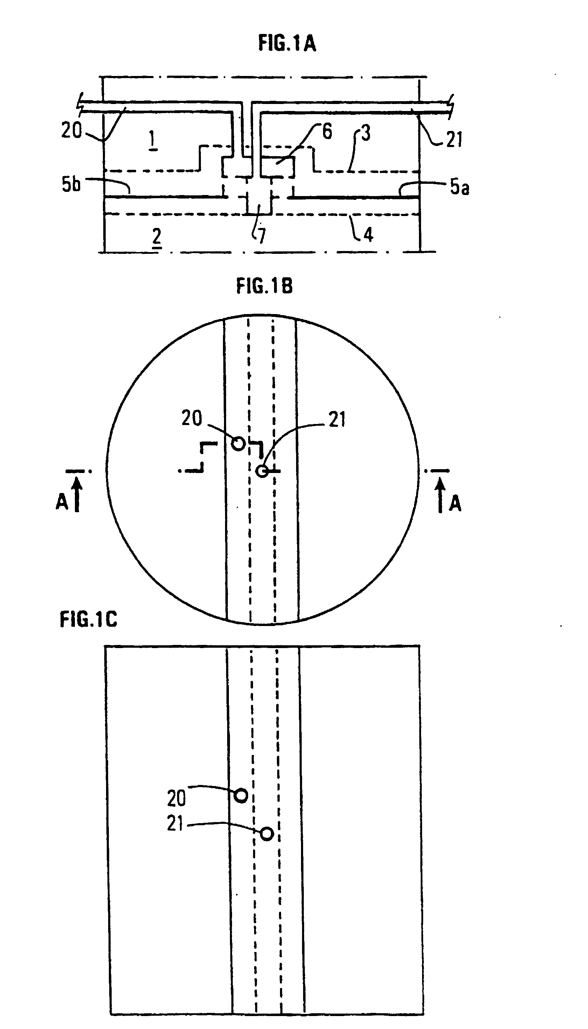

Ces circuits ou rails 6 et 7, sont pourvus sur au moins une de leur paroi d'un ou de plusieurs orifices référencés respectivement 6i et 7i. Sur la figure 1, les orifices 6i sont disposés sur la paroi inférieure du rail 6, et les orifices 7i sur les parois latérales du rail 7. L'axe des orifices 7i sont disposés pour que les flux de fluides percutent l'extrémité des déflecteurs 5a, 5b, par exemple.

Chaque rail est perforé symétriquement et respectivement sur sa face inférieure ou sa face latérale pour le rail supérieur 6, et sur ces faces latérales pour le rail inférieur 7. Des précisions sur la répartition et la dimension des orifices sont explicités ci-après.
* dans le prolongement des déflecteurs 5a et 5b se trouvent disposées des plaques repectivement 8a, 8b, perforées (ouvertures ou fentes 8ai, 8bi). Ces plaques 8a, 8b s'étendent jusqu'aux parois latérales du rail 7 par exemple. Les orifices 8ai, 8bi sont par exemple calibrés pour favoriser le mélange des fluides avant son passage vers l'espace de distribution 12.
* un espace 9 de collecte du fluide principal délimité par la grille supérieure 3, le sommet du rail 6, une paroi latérale du rail 6, les déflecteurs 5a et 5b, (pour des raisons de construction mécanique la variante représentée sur la figure montre une grille en trois parties, les parties étant reliées entre elles par des parois 10a, 10b). Cet espace 9 permet de drainer le fluide principal jusqu'aux chambres de mélanges,
* deux chambres 11a, 11b de mélange disposées de part et d'autre du rail de distribution-collecte 7 ;

Préférentiellement, les chambres sont disposées par exemple par rapport aux orifices 6i, 7i du rail 6 ou 7 ou les deux, qui va ou vont avoir pour fonction d'injecter un ou des fluides dans les chambres de mélange. On les disposera de façon à assurer une injection de fluide la plus homogène, uniforme ou symétrique possible dans l'ensemble de la chambre de mélange par exemple.

La chambre de mélange 11a est par exemple délimitée par une partie de la paroi du rail 6, une paroi latérale du rail 7, le déflecteur 5a et la plaque perforée 8a. La chambre de mélange 11b est délimitée de la même façon par une paroi latérale du rail 7, une partie de la paroi inférieure du rail 6, le déflecteur 5b et la plaque perforée 8b.

Les chambres sont de préférence positionnées au barycentre de la surface du DME.

Le fluide principal collecté par la grille 3 passe de l'espace 9 de collecte vers les chambres de mélange 11a, 11b sous la forme d'une lame de fluide, par la fente formée entre le rail supérieur et la paroi supérieure d'un des déflecteurs.
* un espace 12 de redistribution du mélange issu des chambres de mélange 11a, 11b. Cet espace est délimité par la grille inférieure 4, la paroi inférieure du rail inférieur 7, lorsque celle-ci n'est pas disposée au même niveau que la grille 4 et les chambres de mélange 11a, 11b ainsi que les deux déflecteurs 5a, 5b.

Du fait de l'agencement des orifices, des chambres de mélange et des rails de distribution et/ou de collecte, le mélange obtenu dans l'espace de redistribution présente une composition dont l'homogénéité est améliorée par rapport aux dispositifs de l'art antérieur.

Les rails ou circuits 6, 7 destinés au passage des fluides secondaires, ainsi que les deux chambres de mélange ont par exemple des formes rectangulaires allongées.

Selon une variante de réalisation il est possible de disposer entre l'extrémité inférieure de la paroi 10a, 10b et le déflecteur correspondant 5a, 5b des moyens permettant de créer une série d'orifices calibrés ou des fentes de manière à injecter le fluide principal sous la forme de plusieurs jets dans les chambres de mélange.

Les chambres de mélange peuvent comporter une série d'orifices 8ai, 8bi calibrés dont le but est de favoriser le mélange avant son passage vers l'espace de distribution 12 ou la fonction de la chambre.

La répartition des différents orifices au niveau des rails 6, 7 et de la chambre de mélange est choisie pour que dans la fonction injection, les fluides qui sont injectés vers la chambre de mélange aillent percuter au moins une partie de la paroi pleine d'un des éléments du DME, ce qui favorise le mélange des fluides.

Par exemple, lorsque le rail 6 a une fonction de soutirage et le rail 7 une fonction d'injection, les données géométriques et dimensionnelles pour les orifices 6i, 7i relatifs aux rails de distribution ainsi que leur répartition sur les différentes parois seront par exemple choisies parmi les valeurs suivantes :
* un pas de perforation compris entre 25 et 400 mm et de préférence entre 50 et 200 mm,
* un diamètre compris entre 2 et 15 mm et de préférence dans la gamme 4 à 7 mm,
* une vitesse de passage des fluides compris entre 3 - 20 m/s et de préférence entre 5 - 15 m/s, la vitesse permettant d'alimenter l'ensemble des orifices pour chacun des circuits 6, 7 de la façon la plus homogène possible. La valeur du pas considéré avec la valeur de la vitesse permet d'obtenir un bon mélange du fluide secondaire et du fluide principal.

Les critères donnés pour le choix des valeurs de la vitesse de passage et du pas de perforation restent valables quelque soit la forme des orifices.

La configuration et les critères de dimensionnement du réseau de soutirage sont sensiblement identiques à ceux du réseau d'injection. Une différence peut exister dans le plan de perforation du rail de soutirage. Selon une variante de réalisation, le pas entre les ouvertures du rail de soutirage sera sensiblement égal au double de celui du rail d'injection. Les orifices 6i, 7i seront par exemple disposés en alternance ou en quinconce.

Les ouvertures de sortie 8ai, 8bi de la chambre de mélange ou orifices de passage du mélange auront les caractéristiques suivantes :
* un pas de perforation choisi parmi l'intervalle 25 - 400 mm et de préférence dans l'intervalle 50 - 200 mm,
* un diamètre compris entre 5 et 50 mm, et de préférence entre 10 et 25 mm,
* une vitesse de passage du mélange compris entre 0.5 et 3.5 m/s et de préférence entre 1.0 - 2.0 m/s.

Les orifices ou passage des fluides peuvent présenter tout type de géométrie, tel que une fente unique, plusieurs fentes ou encore des trous.

L'espace de collecte du fluide principal a de préférence une forme adaptée pour minimiser les volumes morts et les turbulences des fluides. Il a une hauteur par exemple comprise entre 3 et 25 mm et de préférence comprise entre 7 et 15 mm une forme sensiblement rectangulaire ou conique. Il peut présenter les caractéristiques mentionnées dans le brevet précité US-A 5.7555.960 du demandeur.

L'espace de redistribution du mélange avant la grille sera par exemple déterminé en tenant compte des caractéristiques physiques du fluide principal.

Le volume de la chambre de mélange sera de préférence adapté pour minimiser les volumes morts. Ses dimensions pourront être choisies parmi celles données dans l'un des brevets précités US-A-5.792.346 ou US-A-5.755.960.

Tout moyen promoteur de turbulence pourra être ajouté à l'intérieur de la chambre de mélange. Ces moyens peuvent se présenter sous la forme de chicanes, de baffles ou tout autre moyen qui sont destinés à accroître l'efficacité du mélange. Le volume de cette chambre sera choisi suffisamment petit pour minimiser l'influence des phénomènes de rétromélange.

La figure 1A schématise une coupe montrant un exemple de disposition d'un DME entre deux lits d'adsorbants 1 et 2 d'une colonne de séparation. Chacun des rails du DME communique avec l'extérieur par l'intermédiaire de conduits 20, 21.

La figure 1B montre une vue de dessus d'un plateau circulaire d'une colonne de séparation comportant un panneau ou DME selon l'invention.

La figure 1C montre une autre variante où le plateau a une forme rectangulaire.

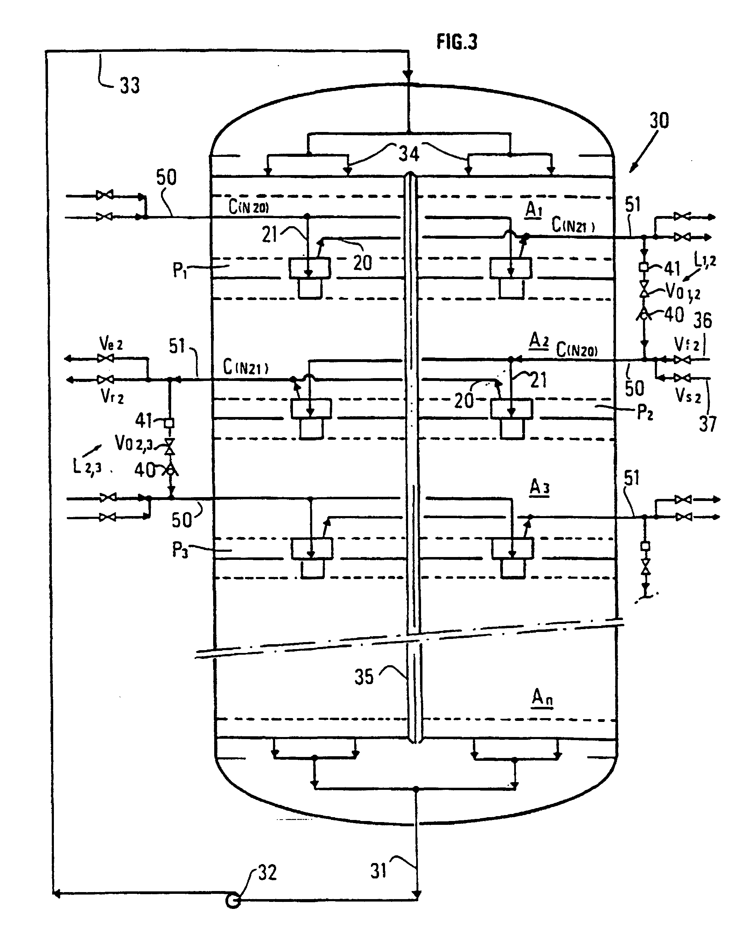

Les figures 2A et 2B schématisent une vue de dessus et une section d'un exemple d'agencement de conduits permettant de mettre en communication un panneau avec l'extérieur de la colonne de séparation.

Cet exemple est donné à titre illustratif et nullement limitatif dans le cas où les rails sont disposés l'un au-dessus de l'autre, l'axe central pour chacun des rails étant sensiblement aligné, le rail supérieur a pour fonction d'extraire le fluide alors que le rail inférieur a pour fonction d'injecter-un fluide dans la chambre de mélange.

Le circuit supérieur 6 utilisé par exemple pour extraire un fluide secondaire, comporte au moins deux conduits 20₁ et 20₂ d'extraction relié à un conduit principal 20. Un conduit 21 permet d'injecter un fluide provenant de l'extérieur de la colonne dans le rail de distribution inférieur 7.

Les deux conduits 20₁ et 20₂ sont disposés de part et d'autre du conduit 21 et de façon telle que le fluide soit injecté de la manière la plus symétrique, la plus homogène possible dans le rail supérieur. Le conduit 21 est de préférence disposé au voisinage de l'axe du rail 6.

Les rails 6 et 7 peuvent assurer différentes fonctions, distribution ou extraction ou encore les deux selon le regroupement des fluides secondaires à l'extérieur de la colonne de séparation dont certains exemples sont donnés ci-après.

Selon une variante de réalisation, un DME ou panneau peut aussi être divisé en plusieurs systèmes d'injection-collecte donc comporter plusieurs systèmes de rails superposés. Dans ce cas les conduits 20, 21 sont divisés de façon à être agencés par rapport aux rails supérieurs et inférieurs d'une manière sensiblement identique à celle décrite aux figures 2A et 2B.

Pour mieux comprendre les avantages donnés par le DME, la figure 3 représente une colonne de séparation par chromatographie en lit mobile simulé qui comporte des panneaux de distribution tels que décrits aux figures 1, 2A, 2B.

La colonne comporte une enceinte 30 par exemple sensiblement cylindrique qui est remplie d'un adsorbant réparti par exemple en plusieurs lits A₁ à Aₙ d'adsorbant, un plateau Pi distributeur de fluide disposé entre chaque lit d'adsorbant, un plateau comportant un ou plusieurs panneaux ou DME.

Du fluide principal est soutiré de l'extrémité inférieure de la colonne par une ligne 31 pour être recyclé par une pompe 32 et une ligne 33 à l'extrémité supérieure de cette colonne où il est introduit dans le lit supérieur A₁ d'adsorbant par des lignes 34. La colonne peut aussi comporter une poutre centrale 35 alignée sensiblement selon l'axe vertical de l'enceinte, par exemple pour des colonnes de grand diamètre.

Sur cet exemple de réalisation, la colonne comporte aussi une ligne de by-pass Li,,j entre les plateaux, dont le principe de fonctionnement est donné dans la demande de brevet FR 2 772 634. Un tel procédé permet notamment d'augmenter la pureté des produits obtenus par un tel procédé.

Pour la séparation de paraxylène à partir d'une charge de xylènes, on dispose par exemple de deux colonnes de douze lits chacune, les vingt-quatre lits étant divisés en au moins quatre zones, chaque zone étant délimitée par une injection d'un fluide de l'extérieur de la colonne (du désorbant ou de la charge par exemple) et un soutirage d'un autre fluide (extrait ou raffinat par exemple). Par exemple, cinq lits sont réservés à la zone I, neuf lits à la zone II, sept lits à la zone III et enfin trois lits à la zone IV.

Les panneaux d'un plateau Pi sont par exemple en communication avec l'extérieur de la colonne par l'intermédiaire de lignes de transfert de fluides secondaires (ligne d'injection 36 de charge, ligne d'injection de désorbant, ligne de soutirage d'un extrait et ligne de soutirage d'un raffinat, et éventuellement une ligne d'injection d'un cinquième fluide de back flush). Les lignes de black flush ne sont pas représentées sur la figure pour des raisons de simplification.

Chacune de ces lignes est équipée d'une vanne séquentielle représentée symboliquement par Vfi, Vei, Vsi et Vri, où l'indice i correspond au plateau Pi et où f désigne la charge, e l'extrait, s le désorbant et r le raffinat. L'ensemble de ces vannes est relié à des moyens de permutation séquentielle adaptés pour faire avancer périodiquement chaque point d'injection de fluide secondaire ou de soutirage de fluide secondaire d'un lit dans le sens de circulation du fluide principal, c'est-à-dire du haut vers le bas de façon à obtenir un fonctionnement en lit mobile simulé.

Le circuit permettant de réaliser le by-pass et d'obtenir une composition d'un fluide sensiblement identique en tous points d'un plateau comporte une ligne de dérivation Li,j reliant deux conduits d'introduction ou de soutirage, et deux plateaux. Une ligne de dérivation comporte selon l'art antérieur, au moins un des dispositifs mentionnés ci-après pris seul ou en combinaison, à savoir un clapet anti-retour 40, un débitmètre 41, une vanne VOi,j de contrôle asservie ou non au débitmètre. Une pompe éventuellement disposée sur la ligne de dérivation peut éventuellement suppléer une insuffisance de perte de charge.

La vanne équipant le by-pass ou ligne de dérivation est référencée V0i.j où l'indice 0 correspond à la fonction de by-pass et les indices i, j les plateaux entre lesquels est effectué le by-pass.

De manière plus générale, un lit mobile simulé comporte au moins quatre zones chromatographiques, avantageusement quatre ou cinq, chacune de ces zones étant constituée par au moins une colonne ou un tronçon de colonne. L'ensemble de ces colonnes ou des tronçons de colonne forme une boucle fermée, la pompe de recyclage entre deux tronçons étant régulée en débit.

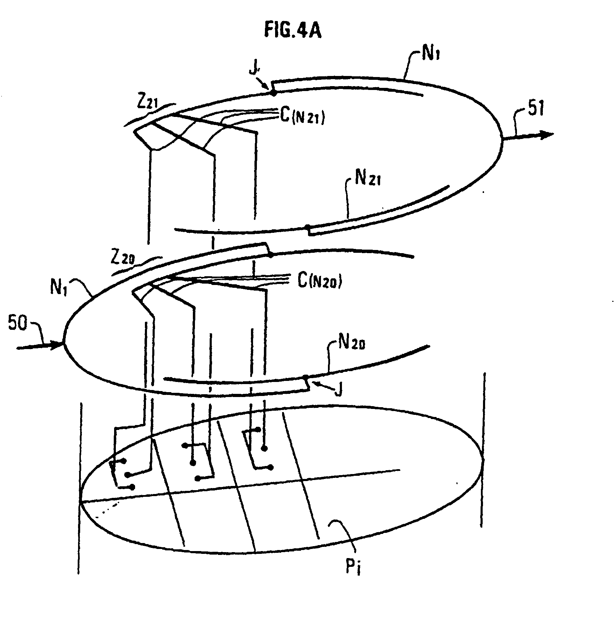

Ces différentes lignes peuvent être en liaison avec un système de distribution-collecte ayant pour fonction de minimiser les dispersions dans les temps de parcours des fluides, et présentant l'une des caractéristiques décrites aux figures 4A à 4C.

Sans sortir du cadre de l'invention tout réseau de distribution ou d'extraction de fluides secondaires habituellement utilisés pour les colonnes de séparation par adsorption peuvent être utilisés.

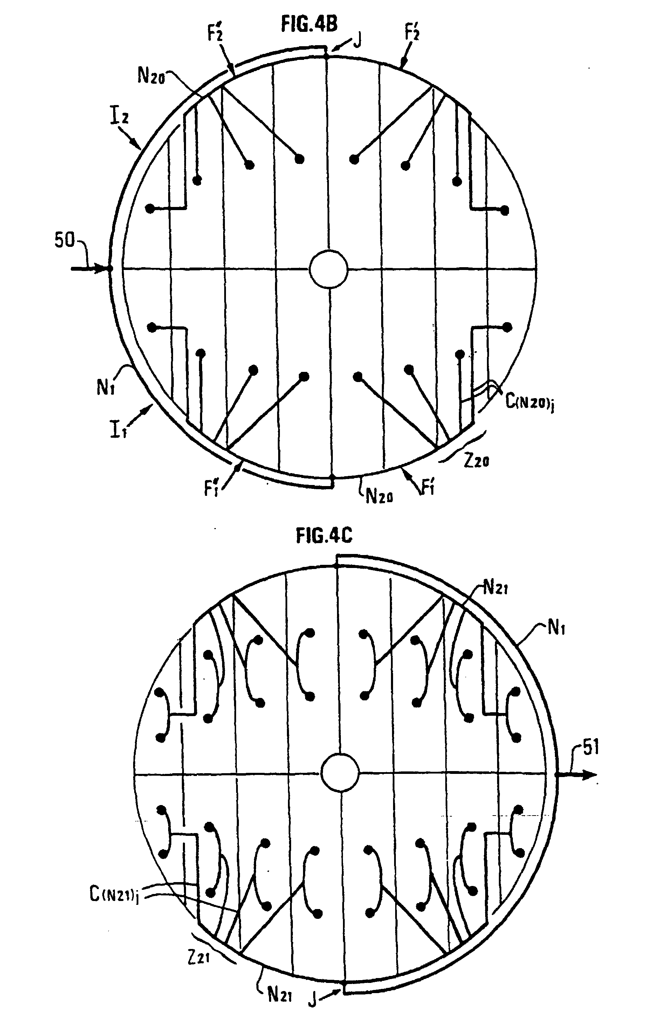

Les figures 4A, 4B et 4C montrent une vue en perspective et des vues en coupe d'un circuit secondaire permettant de distribuer ou d'extraire les fluides secondaires au niveau des différents panneaux pour un plateau donné par exemple. La géométrie et les caractéristiques hydrauliques de ce réseau sont choisies de manière à obtenir une distribution des fluides secondaires la plus symétrique possible pour l'ensemble des panneaux ou des rails de distribution d'un plateau. En particulier cette distribution est choisie pour assurer un indice de déséquilibre faible et diminuer le temps de dispersion par rapport aux systèmes de distribution selon l'art antérieur.

L'exemple qui est donné à titre illustratif et nullement limitatif concerne un système de distribution-collecte adapté pour une colonne sensiblement cylindrique.

Le plateau Pi (figures 4B, 4C) a par exemple la forme d'un disque. Il est découpé en plusieurs DME selon une découpe en parallèles (de type méridien), les panneaux ou DME d'un plateau ayant de préférence des largeurs sensiblement identiques. Une telle découpe assure une densité surfacique de drainage sensiblement constante pour les panneaux.

Pour des plateaux découpés en quatre secteurs, le nombre de DME par secteur est pair de préférence.

Le nombre des systèmes associés à une colonne de séparation est fonction de la géométrie des DME, par exemple.

Sur la variante de réalisation décrite à la figure 4A, un système de distribution-collecte est en liaison avec un des deux rails du DME.
- une chambre annulaire N₁ dite de niveau 1 communique avec l'extérieur de la colonne à l'aide d'un conduit 50 ou 51 par exemple. Ces conduits permettent l'introduction et/ou l'extraction de fluides. Ils peuvent avoir des fonctions précises, injection ou extraction. La chambre annulaire N₁ de section rectangulaire par exemple s'étend en périphérie interne du lit d'adsorbant et assure notamment une division ou une collecte par deux du débit des fluides circulant.
- une chambre annulaire N₂ dite de niveau 2 de section rectangulaire par exemple, est disposée en périphérie de l'adsorbant. Elle assure notamment suivant sa fonction (injection, extraction ou injection/soutirage) respectivement une division ou une collecte ou encore une division et/ou une collecte par deux du débit de fluide.

Sa position suivant la fonction du système de distribution-collecte, en raison de l'encombrement géométrique, peut être par exemple disposée au-dessus (référencée N₂₁) ou en dessous (référencée N₂₀) de la chambre N₁.

La jonction J d'une chambre N₃₀, N₂₁ avec la chambre N₁ de niveau 1 est réalisée par exemple à la moitié de la périphérie de sa longueur.

Une chambre annulaire de niveau 2 comporte, par exemple à chacune de ses extrémités, un ou plusieurs conduits C(N₂₀)j ou C(N₂₁)j de raccordement avec les panneaux d'un plateau, l'indice j correspond à l'indice d'un panneau en liaison avec les conduits. Un conduit C(N₂₀)j ou C(N₂₁)j est raccordé à une zone Z₂₀, Z₂₁ de la chambre annulaire concernée (N₂₁ ou N₂₁).

La longueur d'une chambre annulaire dite de niveau 1 ou N₁ est, par exemple égale à la moitié du périmètre du plateau.

La longueur d'une chambre annulaire dite de niveau 2 ou N₂ est comprise dans un secteur angulaire de 20 à 160 degrés et de préférence entre 100 et 120 degrés.

La zone Z₂₀, Z₂₁ peut être repérée à l'aide d'un angle α compté à partir d'un rayon du plateau, par exemple le rayon passant par le point de jonction J, et par rapport au point central de la zone. La longueur Zr de la zone est déterminée par exemple par le secteur angulaire défini par α +/- ε , borné par les angles αₘᵢₙ et αₘₐₓ.

La valeur de l'angle α sera par exemple prise dans la fourchette (10 degrés, 80 degrés), et de préférence dans la plage (40 degrés, 70 degrés).

La valeur du secteur angulaire sera choisie dans l'intervalle (3 degrés, 30 degrés), et de préférence dans l'intervalle (7 degrés, 15 degrés) ce qui correspond à la longueur Zr des zones Z₂₀ et Z₂₁.

On cherche à avoir une disposition des conduits la plus en étoile possible de manière à obtenir un temps de dispersion le plus faible possible dans la distribution ou l'extraction des fluides. Les points de raccordement des conduites sont regroupés pratiquement en un même point de la zone, dans un secteur angulaire le plus petit possible.

Le positionnement des conduits de raccordement, le choix du secteur angulaire permettent notamment de réduire le temps de retard pur et de dispersion. Ce temps de retard peut ainsi être réduit à 10s.

Chaque conduit C(N₂₀)j, C(N₂₁)j de distribution et/ou d'extraction a un diamètre dj, une longueur lj et un point de raccordement rj avec une chambre de niveau 2 situé dans la zone correspondante Z₂₀, Z₂₁.

Les différents paramètres α, ε , li, sont choisis pour que les fluides distribués arrivent sensiblement en même temps dans tous les panneaux formant un plateau ou dans le cas de l'extraction de fluides parviennent à peu près en même temps jusqu'aux conduits 50, 51.

Le diamètre dj d'un conduit est déterminé en fonction du débit du fluide circulant pour assurer une vitesse de circulation du fluide sensiblement identique dans les différents conduits.

Un conduit peut être en liaison avec un ou plusieurs panneaux DME. Dans ce cas il est prolongé par des ramifications jusqu'à un rail de distribution ou de collecte du panneau.

Les figures 4B et 4C représentent un plateau comportant plusieurs DME, le plateau étant divisé en quatre secteurs selon une découpe en parallèles (de type méridien). Le nombre de panneaux dans ce cas sera de préférence un nombre pair.

La figure 4B montre le système de distribution-collecte qui assure l'injection d'un fluide dans le rail inférieur 7, alors que la figure 4C montre le système de distribution-collecte qui assure le soutirage à partir du rail supérieur 6.

Par exemple en considérant un regroupement des fluides par fonction, la charge et le solvant sont injectés dans un DME alors que l'extrait et le raffinat sont soutirés à partir d'un DME.

### Injection (figures 4A, 4B)

* quatre conduits C(N₂₀)j avec j variant de 1 à 4 permettent l'injection de fluide vers quatre DME d'un secteur quart de plateau,
* la charge et le désorbant sont introduits par le conduit 50 dans la chambre annulaire N₁. Le flux injecté est divisé en deux flux qui parcourent les chemins 11 et 12 et qui correspondent sensiblement à la moitié de la demi-circonférence du plateau. Chaque flux passe ensuite dans la chambre annulaire N₂₀ dans laquelle il est divisé en deux flux F'1 et F"1. Chaque flux est ensuite distribué à partir des conduits de raccordement C(N₂₀)j regroupés dans la zone Z₂₀, au niveau du rail inférieur 7 de chacun des DME.

Les flux issus des orifices 7i vont percuter l'extrémité des déflecteurs 5a, 5b et sont mélangés avec la lame de fluide principal.

### Extraction ou soutirage (figures 4A, 4C)

* quatre conduits C(N₂₁)j vont permettre d'extraire un fluide à partir de 4 panneaux ou DME,
* l'extrait et le raffinat sont soutirés à partir d'un rail d'un panneau, par exemple le rail supérieur 6, et les deux conduits (20₁, 20₂) (figure 2A). Ils passent ensuite à travers les conduits de raccordements C(N₂₁)j en liaison avec la zone Z₂₁ d'une chambre N₂₁. Les deux flux de fluides collectés dans les zones positionnées au niveau des deux extrémités de la chambre annulaire N₂₁ sont regroupés avant de passer dans la chambre annulaire N₁ en liaison avec le conduit 51 qui récupère tous les flux provenant des quatre secteurs du plateau.

Les paramètres donnés pour un DME et pour le système de distribution-collecte ci-dessus à titre indicatif en relation avec une colonne de séparation de forme sensiblement cylindrique pourrait sans sortir du cadre de l'invention être appliquées à une colonne de séparation ayant une forme quelconque, le système de distribution-collecte ayant alors une forme adaptée à celle de la colonne.

De même, un plateau peut être partagé en plusieurs DEM selon une découpe autre que la découpe en parallèles, par exemple en secteurs, de type camembert.

De manière générale, le regroupement des fluides secondaires peut s'effectuer par exemple de trois façons données à titre illustratif et nullement limitatif pour illustrer les connexions de la colonne avec l'extérieur.

En fonction des regroupements des fluides, les rails 6 et 7 assurent des fonctions différentes: une fonction de distribution, une fonction d'extraction ou encore les deux fonctions.

### Cas du regroupement des fluides par fonction :

* Il a été présenté ci-dessus en relation avec les figures 4A, 4B et 4C.

### Cas du regroupement des fluides par nature :

* Les fluides considérés comme fluides propres sont le désorbant et l'extrait et les fluides dits "sales", le raffinat et la charge.
Les rails 6 et 7 assurent dans ce cas les deux fonctions de distribution et d'extraction.

### Cas du regroupement par débit

* Les fluides ayant par exemple un faible débit seront la charge et l'extrait et un débit important le désorbant et le raffinat.
Dans ce cas les rails 6 et 7 assurent les deux fonctions de distribution et d'extraction.

Dans ces deux derniers exemples de regroupement, les fluides injectés par les ouvertures 6i et 7i vont percuter une partie des parois pleines respectivement de la paroi 8 et des déflecteurs 5a, 5b. Ils passent par le système de distribution-collecte en liaison avec le panneau ; alors que les fluides à extraire sont collectés dans les rails 6 ou 7 avant d'être envoyés par les conduits de raccordements vers les chambres annulaires précédemment mentionnées.

Sans sortir du cadre de l'invention les DME et le système de distribution-collecte décrits ci-dessus peuvent aisément être utilisés dans des dispositifs de séparation comportant ou non une poutre centrale de maintien et ayant un diamètre variant par exemple de 3 à 10 m.

Ils peuvent aussi être utilisés pour des colonnes de séparation ayant un diamètre inférieur ou égal à 5m, et ne comportant pas de poutre support centrale.

## Revendications

1. Dispositif (DME) ou panneau pour collecter, distribuer, mélanger ou soutirer plusieurs fluides, au moins un fluide principal et au moins deux fluides secondaires,
comportant au moins :
* des moyens de collecte d'un fluide principal (3, 9),
* au moins deux rails (6, 7) d'injection et/ou de soutirage permettant le passage des fluides secondaires,
* au moins deux chambres de mélange (11a, 11b) qui communiquent avec les rails (6, 7) d'injection et/ou de soutirage à l'aide d'orifices ou des passages (6i, 7i),
* des moyens de redistribution (12, 4) du fluide issu desdites chambres de mélange
* des moyens (5a, 5b) de séparation des moyens de collecte et de redistribution,
caractérisé en ce lesdits rails (6, 7) sont disposés l'un au-dessus de l'autre et en ce que lesdites chambres de mélange (11a, 11b) sont disposées de part et d'autre d'au moins un desdits rails.

2. Dispositif selon la revendication 1 **caractérisé en ce que** lesdites chambres de mélange (11a, 11b) sont disposées par rapport aux orifices (6i, 7i) de manière à obtenir une injection ou un soutirage du ou des fluides la plus uniforme possible dans la ou lesdites chambres de mélange.

3. Dispositif ou panneau selon la revendication 1 **caractérisé en ce que** lesdits rails et lesdites chambres de mélange sont situées sensiblement au barycentre de la surface dudit DME.

4. Dispositif selon l'une des revendications 1 à 3 **caractérisé en ce que** le rail supérieur (6) est pourvu d'au moins deux conduits (20₁, 20₂) et **en ce que** le rail inférieur (7) est pourvu d'au moins un conduit (21), lesdits conduits étant disposés de manière à assurer la circulation des fluides secondaires de la façon la plus homogène dans les rails et les chambres de mélange (11a, 11b).

5. Dispositif selon la revendication 1 **caractérisé en ce que** les ouvertures (6i, 7i) de passage d'un fluide secondaire du ou des rails (6, 7) ayant pour fonction d'injecter des fluides secondaires vers une chambre de mélange (11a, 11b), ont un axe tel que le fluide issu percute une partie au moins d'une paroi pleine d'un des éléments (5a, 5b, 8a, 8b) du DME.

6. Dispositif selon l'une des revendications 1 à 4 **caractérisé en ce que** le circuit inférieur (7) du fluide secondaire a sa paroi inférieure disposée au niveau de la grille inférieure (4).

7. Dispositif ou colonne de séparation comportant une enceinte (30), ladite enceinte comportant au moins un premier lit d'adsorbant (A₁) et au moins un deuxième lit d'adsorbant (A₂), lesdits lits étant séparés par un plateau (Pi) comportant un ou plusieurs panneaux (DME) selon une des revendications 1 à 6, lesdits DME étant en liaison avec l'extérieur par l'intermédiaire de plusieurs conduits de raccordements (20, 21, C(N₂₁), C(N₂₀)).

8. Dispositif selon la revendication 7 **caractérisé en ce que** ledit rail supérieur (6) d'un DME a une fonction de collecte des fluides et ledit rail (7) inférieur d'un DME une fonction d'injection des fluides.

9. Dispositif selon la revendication 7 **caractérisé en ce que** ledit rail supérieur (6) d'un DME a une fonction d'injection des fluides et ledit rail (7) inférieur d'un DME une fonction de collecte des fluides.

10. Dispositif selon la revendication 7 **caractérisé en ce que** lesdits rail supérieur (6) et inférieur (7) d'un DME ont chacun une fonction d'injection-collecte des fluides.

11. Dispositif selon l'une des revendications 6 à 9 **caractérisé en ce que** les ouvertures (6i, 7i) du ou des rails ayant une fonction d'injection sont disposées de façon telles que le jet de fluide passant à travers percute au moins une partie d'une paroi pleine d'un des éléments du DME.

12. Dispositif selon l'une des revendications 7 à 11 **caractérisé en ce que** les ouvertures (6i, 7i) sont disposées en alternance ou en quiconque.

13. Dispositif selon l'une des revendications 7 à 12 **caractérisé en ce que** les paramètres des rails (6, 7) et des ouvertures (6i, 7i) sont choisis parmi les données suivantes:
* un diamètre des ouvertures compris entre 2 et 15 mm et de préférence entre 4 et 7 mm,
* un pas de perforation compris entre 25 et 400 mm et de préférence entre 50 et 200 mm,
* une vitesse de passage des fluides compris entre 3 - 20 m/s et de préférence entre 5 - 15 m/s, la valeur du pas considéré avec la valeur de la vitesse permet d'obtenir un bon mélange du fluide secondaire et du fluide principal.

14. Dispositif selon l'une des revendications 7 à 12 **caractérisé en ce que** les paramètres des chambres de mélange et des ouvertures desdites chambres sont choisis parmi les valeurs suivantes :
* un diamètre des ouvertures (8ai, 8bi) compris entre 5 et 50 mm, et de préférence entre 10 et 25 mm,
* un pas de perforation choisi parmi l'intervalle 25 - 400 mm et de préférence dans l'intervalle 50 - 200 mm,
* une vitesse de passage du mélange compris entre 0.5 et 3.5 m/s et de préférence entre 1.0 - 2.0 m/s.

15. Procédé permettant de séparer au moins un composé à partir d'un mélange ou d'un corps par adsorption **caractérisé en ce que** l'on met en contact un fluide principal à partir duquel on cherche à séparer certains composés avec un adsorbant choisi en fonction de son pouvoir à séparer les composés, on injecte et/ou on extrait les fluides secondaires par l'intermédiaire d'un panneau ou DME pour collecter, distribuer, mélanger ou soutirer plusieurs fluides, au moins un fluide principal et au moins deux fluides secondaires, comportant au moins :
* des moyens de collecte d'un fluide principal (3, 9),
* au moins deux rails (6, 7) permettant le passage des fluides secondaires,
* au moins deux chambres de mélange (11a, 11b) qui communiquent avec les rails (6, 7) d'injection ou de soutirage à l'aide d'orifices ou des passages (6i, 7i),
* des moyens de redistribution (12, 4) du fluide issu desdites chambres de mélange,
* des moyens (5a, 5b) de séparation des moyens de collecte et de redistribution,
les rails (6, 7) étant disposés l'un au-dessus de l'autre et lesdites chambres de mélange disposées de part et d'autre d'au moins un desdits rails.

16. Procédé selon la revendication 15 **caractérisé en ce que** l'on injecte un fluide de manière la plus uniforme possible dans lesdites chambres de mélange en passant par les orifices (6i, 7i) ayant une fonction d'injection.

17. Procédé selon la revendication 15 **caractérisé en ce que** l'on regroupe les fluides par fonction (injection/ou soutirage) ou par nature ou par valeur de débit.

18. Utilisation d'un dispositif selon l'une des revendications 1 à 14 ou du procédé selon l'une des revendications 15 à 17 pour séparer une charge en chromatographie pour des fluides dans un état gazeux, liquide ou supercritique.

19. Utilisation d'un dispositif selon l'une des revendications 1 à 14 ou du procédé selon l'une des revendications 15 à 17 à la séparation de paraxylène en lit mobile simulé.

## Patentansprüche

1. Vorrichtung (DME) oder Paneel zum Sammeln, Verteilen, Mischen oder Umfüllen mehrerer Fluide, mindestens eines Hauptfluids und mindestens zweier Nebenfluide, umfassend mindestens:
◆ Mittel zum Sammeln eines Hauptfluids (3, 9),
◆ mindestens zwei Injektions- und/oder Umfüllschienen (6, 7) zum Durchlaß.der Nebenfluide,
◆ mindestens zwei mit Hilfe von Öffnungen oder Durchlässen (6i, 7i) mit den Injektions- und/oder Umfüllschienen (6, 7) in Verbindung stehende Mischkammern (11a, 11b),
◆ Mittel zur Umverteilung (12, 4) des aus besagten Mischkammem stammenden Fluids,
◆ Mittel (5a, 5b) zur Abgrenzung der Mittel zum Sammeln und zur Umverteilung, **DADURCH GEKENNZEICHNET, daß** die besagten Schienen (6, 7) übereinander angeordnet sind und daß die besagten Mischkammem (11a, 11b) auf beiden Seiten mit mindestens einer der besagten Schienen versehen sind.

2. Vorrichtung nach Anspruch 1, **DADURCH GEKENNZEICHNET, daß** die besagten Mischkammern (11a, 11b) in Bezug auf die Öffnungen (6i, 7i) zur Erzielung einer so homogen wie möglichen Injektion oder Umfüllung des/der Fluids/e in der oder den besagten Mischkammern ausgestaltet sind.

3. Vorrichtung oder Paneel nach Anspruch 1, **DADURCH GEKENNZEICHNET, daß** die besagten Schienen und die besagten Mischkammem im wesentlichen im Schwerpunkt der Oberfläche des besagten DME angeordnet sind.

4. Vorrichtung nach einer der Ansprüche 1 bis 3, **DADURCH GEKENNZEICHNET, daß** die obere Schiene (6) mit mindestens zwei Leitungen (20₁, 20₂)versehen ist und daß die untere Leitung (7) mit mindestens einer Leitung (21) versehen ist, wobei die besagten Leitungen zur Sicherstellung der homogensten Zirkulation der Nebenfluide in den Schienen und den Mischkammern (11a, 11b) ausgestaltet sind.

5. Vorrichtung nach Anspruch 1, **DADURCH GEKENNZEICHNET, daß** die Durchlaßöffnungen (6i, 7i) für ein Nebenfluid der Schiene(n) (6, 7), deren Funktion die Einspritzung der Nebenfluide in eine Mischkammer (11a, 11b) ist, eine solche Achse aufweisen, daß das abgegebene Fluid zumindest einen Teil einer von einem der Elemente (5a, 5b, 8a, 8b) des DME durchdrungenen Wand durchschlägt.

6. Vorrichtung nach einem der Ansprüche 1 bis 4, **DADURCH GEKENNZEICHNET, daß** der untere Kreislauf (7) des Nebenfluids seine untere Wand auf Höhe des unteren Gitters (4) angeordnet hat.

7. Vorrichtung oder Separationskolonne umfassend eine Umgrenzung (30), wobei die besagte Umgrenzung mindestens eine erste Adsorptionsschicht (A₁) und mindestens eine zweite Adsorptionsschicht (A₂) aufweist, wobei die besagten Schichten durch eine Ebene (Pi), welche eine oder mehrere Paneele (DME) nach einem der Ansprüche 1 bis 6 umfaßt, voneinander getrennt sind, wobei die besagten DME vermittels mehrerer Verbindungsleitungen (20, 21, C(N₂₁), C(N₂₀)) mit der Umgebung in Verbindung stehen.

8. Vorrichtung nach Anspruch 7, **DADURCH GEKENNZEICHNET, daß** die besagte obere Schiene (6) eines DME eine Fluid-Sammel-Funktion und die besagte untere Schiene (7) eines DME eine Fluid-Einspritz-Funktion hat.

9. Vorrichtung nach Anspruch 7, **DADURCH GEKENNZEICHNET, daß** die besagte obere Schiene (6) eines DME eine Fluid-Einspritz-Funktion und die besagte untere Schiene (7) eines DME eine Fluid-Sammel-Funktion hat.

10. Vorrichtung nach Anspruch 7, **DADURCH GEKENNZEICHNET, daß** die besagten oberen (6) und unteren (7) Schienen eines DME jeweils eine Einspritz-Sammel-Funktion haben.

11. Vorrichtung nach einem der Ansprüche 6 bis 9, **DADURCH GEKENNZEICHNET, daß** die Öffnungen (6i, 7i) der Schiene(n) mit einer Einspritz-Funktion so angeordnet sind, daß der durchfließende Fluidstrom zumindest einen Teil einer von einem der Elemente des DME durchdrungenen Wand durchschlägt.

12. Vorrichtung nach einem der Ansprüche 7 bis 11, **DADURCH GEKENNZEICHNET, daß** die Öffnungen (6i, 7i) der Schiene(n) abwechselnd oder wechselseitig angeordnet sind.

13. Vorrichtung nach einem der Ansprüche 7 bis 12, **DADURCH GEKENNZEICHNET, daß** die Parameter der Schiene (6, 7) und der Öffnungen (6i, 7i) aus den folgenden Vorgaben ausgewählt sind:
◆ ein Durchmesser der Öffnungen zwischen 2 und 15mm und vorzugsweise zwischen 4 und 7mm liegend,
◆ ein Perforationsschritt zwischen 25 und 400mm und vorzugsweise zwischen 50 und 200mm liegend,
◆ eine Fluiddurchgangsgeschwindigkeit zwischen 3 - 20 m/s und vorzugsweise zwischen 5 - 15 m/s liegend, wobei der Wert des gewählten Schrittes mit dem Wert der Geschwindigkeit es zuläßt, eine gute Durchmischung des Nebenfluids und des Hauptfluids zu erhalten.

14. Vorrichtung nach einem der Ansprüche 7 bis 12, **DADURCH GEKENNZEICHNET, daß** die Parameter der Mischkammer(n) und der Öffnungen der besagten Kammern aus den folgenden Werten ausgewählt sind:
◆ ein Durchmesser der Öffnungen (8ai, 8bi) zwischen 5 und 50 mm liegend, und vorzugsweise zwischen 10 und 25mm liegend,
◆ ein Perforationsschritt aus dem Intervall 25 - 400mm und vorzugsweise aus dem Intervall 50 - 200mm ausgewählt,
◆ eine Durchgangsgeschwindigkeit des Gemisches zwischen 0.5 und 3.5 m/s und vorzugsweise zwischen 1.0 - 2.0 m/s liegend.

15. Verfahren zur Trennung mindestens eines Bestandteils aus einem Gemisch oder aus einem Körper mittels Adsorption, **DADURCH GEKENNZEICHNET, daß** man ein Hauptfluid, aus welcher man bestimmte Bestandteile heraustrennen möchte, mit einem in Abhängigkeit von seiner Trennstärke für Bestandteile ausgewählten Adsorber zusammenbringt, daß man die Nebenfluide mittels einer Paneele oder DME, um mehrere Fluide - mindestens ein Hauptfluid und mindestens zwei Nebenfluide - zu sammeln, zu verteilen, zu mischen oder abzufüllen, injiziert und/oder abscheidet, umfassend mindestens:
◆ Mittel zum Sammeln eines Hauptfluids (3, 9),
◆ mindestens zwei Schienen (6, 7), die den Durchlaß der Nebenfluide zulassen,
◆ mindestens zwei Mischkammern (11 a, 11 b), die mit den Injektions- oder Umfüllschienen (6, 7) mit Hilfe von Öffnungen oder Durchlässen (6i, 7i) in Verbindung stehen
◆ Mittel zur Umverteilung (12, 4) des aus besagten Mischkammern stammenden Fluids,
◆ Mittel (5a, 5b) zur Abgrenzung der Mittel zum Sammeln und zur Umverteilung, wobei die Schienen (6, 7) übereinander angeordnet sind und die besagten Mischkammern auf beiden Seiten mit mindestens einer der besagten Schienen versehen sind.

16. Verfahren nach Anspruch 15, **DADURCH GEKENNZEICHNET, daß** man ein Fluid so homogen wie möglich in die besagten Mischkammem durch die Öffnungen (6i, 7i), die der Injektion dienen, injiziert.

17. Verfahren nach Anspruch 15, **DADURCH GEKENNZEICHNET, daß** man die Fluide in Abhängigkeit von der Funktion (Injektion/oder Umfüllung) oder nach Eigenschaft oder nach Durchsatzwert umgruppiert.

18. Verwendung einer Vorrichtung nach einem der Ansprüche 1 bis 14 oder des Verfahrens nach einem der Ansprüche 15 bis 17, um eine Bestückung in Chromatographie zu trennen für Fluide in gasförmigem, flüssigem oder überkritischem Zustand.

19. Verwendung einer Vorrichtung nach einem der Ansprüche 1 bis 14 oder des Verfahrens nach einem der Ansprüche 15 bis 17 zur Trennung von Paraxylen in simulierter mobiler Schicht.

## Claims

1. Device (DME) or panel for collecting, distributing, mixing or drawing off several fluids, at least one main fluid and at least two secondary fluids that, comprise at least:
• means for collecting a main fluid (3, 9),
• at least two injection and/or draw-off rails (6, 7) that allow the passage of secondary fluids,
• at least two mixing chambers (11a, 11b) that communicate with injection and/or draw-off rails (6, 7) with orifices or passages (6i, 7i),
• means (12, 4) for redistributing the fluid that is obtained from said mixing chambers,
• means (5a, 5b) for separating collecting and redistributing means, **characterized in that** said rails (6, 7) are arranged one above the other, and **in that** said mixing chambers (11a, 11b) are arranged on both sides of at least one of said rails.

2. Device according to claim 1, wherein said mixing chambers (11a, 11b) are arranged relative to orifices (6i, 7i) to obtain an injection or a draw-off of the fluid or fluids that is the most uniform possible in said mixing chamber(s).

3. Device or panel according to claim 1, wherein said rails and said mixing chambers are located approximately in the center of gravity of the surface of said DME.

4. Device according to one of claims 1 to 3, wherein upper rail (6) is provided with at least two hoses (20₁, 20₂) and wherein lower rail (7) is provided with at least one hose (21), whereby said hoses are arranged to ensure the circulation of secondary fluids in the most homogeneous way in the rails and mixing chambers (11a, 11b).

5. Device according to claim 1, wherein openings (6i, 7i) for passage of a secondary fluid from rail(s) (6,7) whose function is to inject secondary fluids into a mixing chamber (11a, 11b) has an axis such that the fluid that is obtained strikes at least a portion of a solid wall of one of elements (5a, 5b, 8a, 8b) of the DME.

6. Device according to one of claims 1 to 4, wherein lower circuit (7) of the secondary fluid has its lower wall arranged at lower grid (4).

7. Device or separation column that comprises a chamber (30), whereby said chamber comprises at least a first adsorbent bed (A₁) and at least a second adsorbent bed (A₂), whereby said beds are separated by a plate (Pi) that comprises one or more panels (DME) according to one of claims 1 to 6, whereby said DME are linked with the outside via several connecting hoses (20, 21, C(N₂₁),C(N₂₀)).

8. Device according to claim 7, wherein said upper rail (6) of a DME has a function of collecting fluids, and said lower rail (7) of a DME has a fluid injection function.

9. Device according to claim 7, wherein said upper rail (6) of a DME has a function of injecting fluids, and said lower rail (7) of a DME has a function of collecting fluids.

10. Device according to claim 7, wherein said upper rail (6) and lower rail (7) of a DME each have a fluid injectingcollecting function.

11. Device according to one of claims 6 to 9, wherein openings (6i, 7i) of the rail or rails that have an injection function are arranged such that the fluid jet that passes through strikes at least a portion of a solid wall of one of the elements of the DME.

12. Device according to one of claims 7 to 11, wherein openings (6i, 7i) are arranged alternately or at random.

13. Device according to one of claims 7 to 12, wherein the parameters of rails (6, 7) and openings (6i, 7i) are selected from among the following data:
• a diameter of the openings of between 2 and 15 mm and preferably between 4 and 7 mm,
• a perforation span of between 25 and 400 mm and preferably between 50 and 200 mm,
• a rate of flow of the fluids of between 3-20 m/s and preferably between 5-15 m/s, and the value of the span that is being considered with the value of the rate makes it possible to obtain a good mixing of the secondary fluid and the main fluid.

14. Device according to one of claims 7 to 12, wherein the parameters of the mixing chambers and the openings of said chambers are selected from among the following values:
• a diameter of openings (8ai, 8bi) of between 5 and 50 mm, and preferably between 10 and 25 mm,
• a perforation span that is selected from the interval of 25-400 mm and preferably in the interval 50-200 mm,
• a rate of flow of the mixture of between 0.5 and 3.5 m/s and preferably between 1.0-2.0 m/s.

15. Process that makes it possible to separate at least one compound from a mixture or an element by adsorption, wherein a main fluid, from which it is sought to separate some compounds, is brought into contact with an adsorbent that is selected as a function of its ability to separate the compounds, and the secondary fluids are injected and/or extracted via a panel or DME to collect, distribute, mix or draw-off several fluids, at least one main fluid and at least two secondary fluids that comprises at least:
• means for collecting a main fluid (3, 9),
• at least two rails (6, 7) that allow the passage of secondary fluids,
• at least two mixing chambers (11a, 11b) that communicate with injection and/or draw-off rails (6, 7) with orifices or passages (6i, 7i),
• means for redistributing (12, 4) the fluid that is obtained from said mixing chambers,
• means (5a, 5b) for separating collecting and redistributing means, whereby rails (6, 7) are arranged one above the other, and said mixing chambers are arranged on both sides of at least one of said rails.

16. Process according to claim 15, wherein a fluid is injected in the most uniform manner possible into said mixing chambers by passing through orifices (6i, 7i) that have an injection function.

17. Process according to claim 15, wherein the fluids are grouped by function (injection or draw-off) or by nature or by flow rate value.

18. Use of a device according to one of claims 1 to 14 or of the process according to one of claims 15 to 17 to separate a feedstock by chromatography for fluids in a gaseous state, liquid state or supercritical state.

19. Use of a device according to one of claims 1 to 14 or of the process according to one of claims 15 to 17 with the separation of paraxylene in a simulated moving bed.
